(19) Europäisches Patentamt
European Patent Office
Office européen des brevets

(11) Veröffentlichungsnummer : **0 339 420 B1**

# (12) EUROPÄISCHE PATENTSCHRIFT

(45) Veröffentlichungstag der Patentschrift : **04.03.92 Patentblatt 92/10**

(51) Int. Cl.$^5$ : **A61K 9/24,** A61K 9/36, A61K 31/44

(21) Anmeldenummer : **89106822.3**

(22) Anmeldetag : **17.04.89**

## (54) DHP-Retard-Zubereitung.

(30) Priorität : **29.04.88 DE 3814532**

(43) Veröffentlichungstag der Anmeldung : **02.11.89 Patentblatt 89/44**

(45) Bekanntmachung des Hinweises auf die Patenterteilung : **04.03.92 Patentblatt 92/10**

(84) Benannte Vertragsstaaten : **AT BE CH DE ES FR GB GR IT LI NL SE**

(56) Entgegenhaltungen :
**EP-A- 0 047 899**
**EP-A- 0 142 561**
**EP-A- 0 306 699**
**PHARMAZEUTISCHE INDUSTRIE, Band 41, Nr. 8, 1979, Seiten 799-802, Cantor Verlag, Aulendorf, DE; J.L. SALOMON et al.: "Sustained release of a water-soluble drug from hydrophilic compressed dosage forms"**

(73) Patentinhaber : **BAYER AG**
**W-5090 Leverkusen 1 Bayerwerk (DE)**

(72) Erfinder : **Ohm, Andreas, Dr.**
**Am Röttgen 42**
**W-4040 Neuss 1 (DE)**
Erfinder : **Luchtenberg, Helmut, Dr.**
**Windmühlenstrasse 11a**
**W-5216 Niederkassel (DE)**
Erfinder : **Bücheler, Manfred, Dipl.-Ing.**
**Lorkenhöhe 49**
**W-5063 Odenthal 2 (DE)**
Erfinder : **Rupp, Roland, Dr.**
**Solinger Strasse 18**
**W-5653 Leichlingen 2 (DE)**
Erfinder : **Feltkamp, Heinrich, Dr.**
**Höhenstrasse 10**
**W-5202 Hennef (DE)**

EP 0 339 420 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft feste Arzneizubereitungen mit lang anhaltender Wirkung für Dihydropyridine sowie Verfahren zu ihrer Herstellung, bestehend aus einem Kern sowie einem darum angebrachten Mantel.

Wirkstoffe aus der Stoffklasse der Dihydropyridine und ihre Verwendung als Kreislaufmittel sind bereits bekannt (vgl. Brit. Pat. 1 173 862, Brit. Pat. 1 358 951, US-Pat. 4 256 749, DE-OS 3 311 003 and US-Pat. 4 264 611). Bei der galenischen Zubereitung dieser potenten Wirkstoffe treten häufig Schwierigkeiten auf, da die Stoffe nur eine sehr geringe Löslichkeit besitzen, häufig lichtempfindlich sind und ihre Resorbierbarkeit in biologischen Systemen häufig zu Problemen führt.

Es wurden zahlreiche Versuche unternommen, optimale galenische Zubereitungen herzustellen, die die Bioverfügbarkeit dieser potenten Wirkstoffe verbessert. So wurden z.B. einige Wirkstoffe in speziellen organischen Lösungsmittelsystemen gelöst und in Gelatinekapseln eingefüllt, um einen schnellen und effektiven Wirkungseintritt zu gewährleisten (vgl. Brit. Pat. 1 362 627). Es wurde auch versucht, Dihydropyridine wie Nifedipin unter Verwendung von wasserlöslichen Polymeren in Copräzipitate bzw. "feste Lösungen" zu überführen, um die Bioverfügbarkeit zu verbessern (vgl. Brit. Pat. 1 579 818). Mit diesen galenischen Zubereitungsformen kann jedoch bei den genannten Dihydropyridinen i.a. keine Reduktion der Einnahme auf 1 bis maximal 2 mal täglich erreicht werden.

Für die Behandlung von Krankheiten, die über längere Zeiträume behandelt werden müssen, wie z.B. Hypertonie und Angina pectoris ist es wünschenswert, die Häufigkeit der Einnahme von Medikamenten so gering wie möglich zu halten. Dies ist nicht nur angenehmer für den Patienten, sondern es erhöht auch die Behandlungssicherheit, indem es die Nachteile unregelmäßiger Einnahmen vermindert und die vorhandene Wirkstoffkonzentration im Körper stabilisiert. Dadurch wird gleichzeitig das Risiko von unerwünschten Über- bzw. Unterdosierungen minimiert (Blutspiegelspitzen nach Einnahme schnell den Wirkstoff freisetzender Darreichungsformen bzw. unregelmäßige oder vergessene Einnahme bei häufiger Applikationsfrequenz).

Von besonderem Vorteil sind Darreichungsformen, die den Wirkstoff entsprechend dem Bedarf des Patienten abgeben.

So ist z.B. für den Verlauf des Blutdrucks (Lemmer, B. in: Chronopharmakologie, Tagesrhythmen und Arzneimittelwirkung, Wiss. Verl. GmbH, Stuttgart 1984) ein Tagesrhythmus beschrieben: während der Nacht sinken sowohl normo- als auch hypertone Werte (systolisch und diastolisch) auf ein Minimum ab (gegen 04.00 Uhr), um daraufhin in den frühen Morgenstunden (auch während des Schlafes) steil wieder ansteigen (Maximum des Blutdrucks gegen 10.00 Uhr). Ein sich dem Tagesrhythmus anpassendes Blutdruckmedikament hätte demzufolge gegenüber konventionellen retardierten Systemen den Vorteil, den Patienten nur dann mit Wirkstoff zu belasten, wenn Wirkstoff benötigt wird. Insbesondere kann der steile Blutdruckanstieg in den frühen Morgenstunden trotz des Schlafes des Patienten durch Abgabe überproportional hoher Mengen Wirkstoff sicher abgefangen werden, ohne den Körper in den Zeiten mit Blutdruckabfall mit dann nicht benötigtem Wirkstoff zu belasten (Beispiel: abendliche (nächtliche) Einnahme, Placebophase während des Blutdruckabfalls, Beginn Wirkstofffreisetzung in den frühen Morgenstunden).

Ähnliches gilt auch für die Angina pectoris: Bei Patienten mit einer stabilen Angina pectoris oder mit einer Prinzmetal-Angina pectoris sind unter körperlicher Belastung tageszeitabhängige Unterschiede im EKG und in der Anfallshäufigkeit nachzuweisen. Epidemiologische Studien haben gezeigt, daß auch die Häufigkeitsverteilung an Herzinfarkt-Anfällen einem Tagesrhytmus unterliegen. So weist die kardiale Morbidität in den frühen Morgenstunden ein Maximum auf.

Sowohl für den Arzt als auch für den Patienten besteht ein Bedürfnis, z.B. für die Dauertherapie von Herzkreislauferkrankungen, die hochwirksamen Dihydropyridine in einer Form zur Verfügung gestellt zu bekommen, daß eine einmal tägliche Applikation zur Krankheitsbehandlung ausreicht. Für Dihydropyridine wurden bereits Arzneizubereitungen mit verzögerter Wirkstoff-Freigabe (Retard-Formen) beschrieben. So wurde z.B. versucht, durch eine spezielle Korngrößenverteilung des kristallinen Wirkstoffs bzw. durch eine ausgewählte spezifische Oberfläche der Wirkstoffkristalle eine langsam freisetzende Zubereitung herzustellen (vgl. DE-OS 3 033 919). Weiterhin wurden spezielle Tablettenzubereitungen vorgeschlagen, die nach dem Prinzip der osmotischen Pumpe den Wirkstoff aus dem Inneren einer Tablette, die mit einer semipermeablen Lackschicht umgeben ist, durch eine vorgegebene Öffnung über einen längeren Zeitraum freisetzen und somit einen Retard-Effekt erzielen (vgl. US-PS 3 916 899).

Die bisher bekannten Zubereitungsformen mit verzögerter Wirkstoffabgabe, insbesondere solche für Dihydropyridine, weisen eine Reihe von Nachteilen auf. Ihre Retard-Wirkung ist nur auf einige Stunden beschränkt, so daß der Patient in der Regel nach wie vor zwei- oder mehrmals täglich applizieren muß: Nach einigen Stunden läßt die Freisetzungsgeschwindigkeit des Wirkstoffs deutlich nach, so daß auch die Blutspiegel unter die erforderliche Effektivitätsgrenze absinken können.

Bei dem oben erwähnten osmotischen System kann es im Magen oder Darmtrakt je nach eingesetzter Kapselfüllung zu lokalen Irritationen des Gewebes durch überhöhte Konzentration an Wirkstoff kommen.

Bedingt durch die Natur des osmotischen Systems kann ein Teil des Wirkstoffs in der Arzneiform verbleiben und somit nicht für die gewünschte Resorption zur Verfügung stehen. Die Herstellung dieser Arzneiform ist zudem sehr aufwendig, da hierbei organische Lösungsmittel im Herstellungsprozeß eingesetzt werden müssen und die Lackschicht jeder Tablette einzeln mit Hilfe eines Laserstrahls durchbohrt werden muß.

In allen bisher beschriebenen Retardarzneiformen ist eine Anpassung der Wirkstofffreisetzung an tagesrhythmische biologische Geschehnisse (z.B. Blutdruck) und pathologische Ereignisse (pathologische EKG-Veränderungen, Angina-pectoris-Anfälle) nicht möglich (bei Einmalgabe). Eine Lackierung mit magensaftresistenten Überzügen, die bewirkt, daß ein Teil oder die gesamte Dosis erst nach Verlassen des Magens aus der Arzneiform freigesetzt wird (diskontinuierlich freisetzende Retardform), ist schon aufgrund der pH-Abhängigkeit der Wirkstoffliberation unzuverlässig, da die Magenverweilzeiten von Patient zu Patient extrem streuen und zudem von der Nahrungsmittelaufnahme abhängen (Magenverweilzeiten ca. 0,2-12 h). Mit einer solchen Arzneiform kann zwar ein Teil des Arzneistoffes zeitlich versetzt freigesetzt werden, die Reproduzierbarkeit dieser Verzögerungszeit (lag time) ist allerdings aus o.g. Gründen nicht gegeben, so daß z.B. eine sichere Anpassung an tagesrhythmusabhängige biologische Prozesse damit nicht erreicht werden kann.

Es wurde nun gefunden, daß feste Arzneizubereitungen mit langanhaltender Wirkung, bestehend aus einem Kern und einem darumgelegten Mantel, die einen schwer löslichen Dihydropyridin-Wirkstoff insbesondere der allgemeinen Formel I

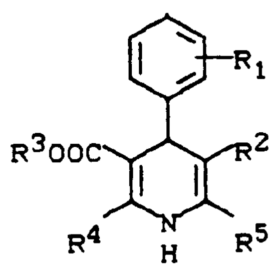

I,

enthalten, in welcher

$R^1$ für ein oder zwei gleiche oder verschiedene Substituenten aus der Gruppe Nitro, Halogen, Trifluormethyl oder $OCHF_2$ steht oder in der

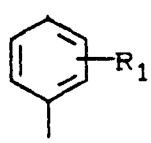

für

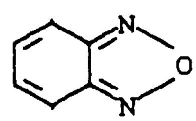

oder

für

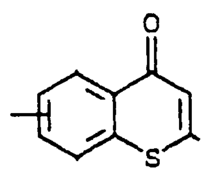

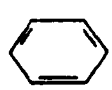

steht,

$R^2$ für eine Nitrogruppe steht oder für den Rest $COOR_6$ steht, wobei

$R_6$ Alkyl mit 1 bis 10 C-Atomen bedeutet, welches gegebenenfalls substituiert ist durch Alkoxy mit 1 bis 4 C-Atomen oder durch ein oder mehrere Halogene

oder wobei

$R^2$ gemeinsam mit $R^5$ für die Lactongruppe $-CO-O-CH_2-$ steht,

$R^3$ für Alkyl mit 1 bis 10 C-Atomen steht, welches gegebenenfalls substituiert ist durch Alkoxy mit 1 bis 4 C-Atomen oder durch ein oder mehrere Fluore und

$R^4$ und $R^5$ gleich oder verschieden sind und jeweils für Alkyl mit 1 bis 4 C-Atomen, welches gegebenenfalls durch Hydroxy substituiert ist, stehen,

wobei die Kern-Mantel-Darreichungsform

a) aus einem Kern besteht, der mindestens eins der obengenannten Dihydropyridine in langsam freisetzender Form enthält und

b) aus einem um den Kern liegenden Manten besteht, der keinen Wirkstoff enthält und der sich nur langsam

auflöst, und

c) auf dem Mantel gegebenenfalls zusätzlich eine schnell freisetzende Initialdosis des Wirkstoffes aufgebracht ist,

eine überraschende Effektivität zeigen.

Bevorzugt seien solche Zubereitungen genannt, die im Kern 10 % bis 100 %, vorzugsweise 50 % bis 100 % des gesamten Dihydropyridin-Wirkstoffs der Darreichungsform enthalten.

Je nach Art des Wirkstoffs enthalten die erfindungsgemäßen Zubereitungen insgesamt vorzugsweise 1 bis 200 mg, insbesondere 10 bis 150 mg mindestens eines Wirkstoffs aus der Klasse der Dihydropyridine.

Der langsam freisetzende Kern der Zubereitung enthält den Wirkstoff vorzugsweise in fein gemahlener oder mikronisierter kristalliner Form.

Als Kern mit langsamer Freisetzung werden vorzugsweise solche Kerne verstanden, die den Wirkstoff in retardierter Form enthalten und ihn zu weniger als 75 % in einer Zeit von einer Stunde freisetzen / Prüfbedingungen entsprechend der Spezifikation für feste Formulierungen der Wirkstoffe, z.B. für Nifedipin, Nitrendipin, Nimodipin und Nisoldipin: nach einer der unten genannten Bedingungen:

Bedingung A: USP-Paddle, 4 l künstlicher Magensaft ohne Pepsin plus 0,1 % Tween 80, 100 Upm, 37°C,
Bedingung B: USP-Paddle, 0,9 l 0,1 N-Salzsäure plus 0,25 % Texapon K12, 100 Upm, 37°C,
Bedingung C: USP-Paddle, 0,9 l Methanol/Wasser (40:60) 50 Upm, 37°C.

Die Retardierung der Kernfreisetzung kann nach den üblichen Methoden erfolgen (z.B. Nifedipin nach EP-B 47 899) oder anderen dem Stand der Technik entsprechenden Methoden.

Der Mantel enthält keinen Wirkstoff. Im Gemisch mit pharmazeutisch üblichen Hilfsstoffen wie z.B. Laktose, Stärke, Cellulose, Citronensäure u.a. und Magnesiumstearat als Schmiermittel bildet ein hydrophiles gelbildendes Polymer das Mantelmaterial. Dieses hydrophile Polymer steuert die Auflösungsgeschwindigkeit und Erosion des Mantels. Steuerungsfaktoren für die Ablösungs/Erosionsgeschwindigkeit des Mantelmaterials sind u.a. die Schichtdicke des Mantels und das Verhältnis Polymer(e)/restliche Hilfsstoffe.

Als hydrophile gelbildende Polymere sind z. B. modifizierte Stärke und/oder celluloseartige Substanzen wie Methylcellulose, Hydroxypropylmethylcellulose, Hydroxypropylcellulose und Natriumcarboxymethylcellulose geeignet.

Die Erosions- und Auflösungsgeschwindigkeit des Mantels kann auch gesteuert werden über die unterschiedlichen Viskositätsgrade des Polymeren, wobei die Geschwindigkeit sich erhöht, wenn niederviskose Qualitäten eingesetzt werden und langsamer wird beim Einsatz hochviskoser Typen. Üblicherweise liegt die Konzentration des Polymeren im Mantelmaterial bei 5 - 100 %, vorzugsweise bei 25 - 90 %. Die eingesetzte Konzentration ist abhängig vom Viskositätsgrad des(r) Polymeren und von der Löslichkeit/Hydrophilie der eingesetzten Hilfsstoffe und deren Menge.

Übliche bekannte galenische Maßnahmen wie z.B. das Lackieren des Kerns mit einer magensaftresistenten Schicht oder anderen Lacken, die Verwendung von Geschmacks- und Aromastoffen und Schmiermitteln und üblichen Hilfsmitteln, die dem galenischen Fachmann geläufig sind, können selbstverständlich auch bei der erfindungsgemäßen Zubereitung eingesetzt und verwendet werden.

Geeignete feste Darreichungsformen für das erfindungsgemäße Kern-Mantel-Prinzip sind z.B. Pellets und Manteltabletten. Die Größe der Zubereitung kann von 0,5 - 15 mm variieren.

Ein besonderer Vorteil bei der Verwendung von Pellets liegt in der Teilung der Gesamtwirkstoffdosis in mehrere Untereinheiten (sog. multiple dose), die es erlaubt, Pellets mit verschieden dicken oder mit verschieden zusammengesetzten Überzügen so zu kombinieren, daß nahezu jedes gewünschte Freisetzungsprofil der die Gesamtdosis enthaltenden Darreichungsform (z. B. Kapsel) eingestellt werden kann, z. B. eine lineare Wirkstoffabgabe oder eine kontinuierlich schneller werdende Freisetzung des Wirkstoffes oder eine Abgabe des Wirkstoffes in Stößen. Die Initialdosis kann hier z. B. durch nicht ummantelte Wirkstoffkerne eingebracht werden.

Im Fall von Manteltabletten kann die Initialdosis auf den Placebomantel aufgebracht werden, so daß hier eine diskontinuierliche, stoßweise Freisetzung erzielt wird.

Die eingestellte Verzögerungszeit bis zur Freisetzung des Wirkstoffes im Kern durch Aberosion des Mantelmaterials bedingt den gewünschten Retardeffekt. Die Verzögerungszeit durch Aberosion/Ablösen des Mantels wird dabei vom pH nicht entscheidend beeinflußt.

Es sei ausdrücklich darauf hingewiesen, daß die erfindungsgemäße Retard-Zubereitung sich von den bisher bekannten Kern-Mantel-Zubereitungen dadurch unterscheidet, daß der Mantel keinen Wirkstoff enthält.

Die Kerne der erfindungsgemäßen Formulierungen werden hergestellt durch bekannte Methoden, z. B. durch Mischen von Nifedipin-Kristallen mit einer spezifischen Oberfläche von 1 - 4 $m^2/g$ und geeigneten Trägerstoffen, wie in EP-B 47 899 beschrieben.

Aus dem Stand der Technik sind bereits Mehrschicht-Tabletten auf Basis von Casein-Matrices beschrieben, die zwei oder drei Schichten enthalten, die jeweils ihrerseits Wirkstoffe enthalten können (vgl. US-Pat. 3

184 386). Die dort beschriebenen Tabletten enthalten im Gegensatz zur vorliegenden Erfindung im äußeren Manten Wirkstoff.

Auch in der US-Pat. 3 558 768 werden Manteltabletten beschrieben, die sowohl im Kern als auch im Mantel Wirkstoffe in langsam freisetzender Form enthalten.

Manteltabletten, die im Mantelmaterial keinen Wirkstoff enthalten, sind auch in Il Farmaco, No 3, März 84, 67 f (Conte et al.) beschrieben. Die Freisetzungskinetik dieser Tabletten unterscheidet sich allerdings deutlich von dem hier beschriebenen erfindungsgemäßen Kern-Mantelprinzip: nach einer Verzögerungszeit wird der Wirkstoff über lange Zeit nach einer Kinetik nullter Ordnung kontinuierlich freigesetzt. Das Mantelmaterial dient hier als Diffusionsbarriere, nicht aber zur Einstellung einer diskontinuierlich erfolgenden Freisetzung des Wirkstoffes. Im Gegensatz zum vorliegenden erfindungsgemäßen Kern-Mantel-Prinzip kann die "Reservoirmanteltablette" nur bei Wirkstoffen mit einer gewissen Mindestwasserlöslichkeit eingesetzt werden.

Auch Salomon et al. (Pharm. Ind. 41. Nr. 8, S 799 f. 1979) beschreiben Manteltabletten, die keinen Wirkstoff im Mantelmaterial enthalten. Auch hier handelt es sich um eine "Reservoirmanteltablette". Im Prinzip gilt das oben Gesagte.

Das in den vorgenannten Beispielen beschriebene Diffusionsprinzip ist für schwerlösliche Dihydropyridine nicht geeignet. Ein auf einem Diffusionsprinzip beruhendes Retardierungsverfahren führt im Fall der schwerlöslichen Dihydropyridine zu extrem langsamer Freisetzung des Wirkstoffes und daraus resultierend zu vergleichsweise niedrigen Pharmaspiegeln.

Eine weitere Darreichungsform, die speziell den sättigbaren first pass-Effekt von Psoralenen günstig beeinflußt, ist in DE 3 115 033 A 1 beschrieben. Das Mantelmaterial enthält hierbei allerdings Wirkstoff. Zudem wird die Verzögerung bis zur Freisetzung von Wirkstoff aus dem Kern der Manteltabletten bzw. Pellets nicht durch den Auftrag hoher Anteile an hydrophilen, gelbildenden Polymeren im Mantel, sondern durch eine Lackierung des Kerns/ Pellets erreicht (ein dünner Lackfilm).

In der DE-OS 2 651 176 werden Pellets mit kontrollierter Wirkstoffabgabe beschrieben. Die dort genannten Formulierungen unterscheiden sich von den erfindungsgemäßen Mantelzubereitungen schon dadurch, daß sie auch im Mantel Wirkstoff enthalten. Zudem können die dort beschriebenen Formulierungen nur in aufwendigen Verfahren durch kontinuierliches Aufbringen vieler Schichten erhalten werden, während die erfindungsgemäße Manteltablette durch einfaches Verpressen hergestellt wird und im Fall der Mantelpellets nur eine Schicht kontinuierlich auf die langsam freisetzenden Kerne aufgebracht wird.

Durch das Prinzip der erfindungsgemäßen Zubereitung werden die üblichen Nachteile von normalen Retard-Tabletten bzw. -Pellets und auch von bisher bekannten Mehrschicht- oder Manteltabletten und Pellets bzw. von Zubereitungsformen die auf dem osmotischen Prinzip beruhen, vermieden.

Insbesondere kann durch die erfindungsgemäße Manteldarreichungsform eine Anpassung der Wirkstofffreisetzung an tagesrhythmusabhängige biologische Prozesse, wie z.B. den Blutdruck, erfolgen, ohne die Form zu unüblichen Tageszeiten applizieren zu müssen. Als ein weiterer Vorteil dieser Form ist zu nennen, daß bei Wirkstoffen, die bei Resorption in tiefen Abschnitten des Magen-Darm-Trakts eine erhöhte Bioverfügbarkeit im Vergleich zur Resorption im Magen zeigen, diese Darreichungsform zu einer verbesserten Verfügbarkeit des applizierten Wirkstoffs führt. Betont werden muß die diskontinuierliche durch die Erosion des Mantels bedingte Freisetzung aus dieser Retardform, die zudem pH-unabhängig erfolgt. Dadurch können auch die schwerlöslichen erfindungsgemäßen Dihydropyridine in geeigneter Weise retardiert werden.

Im Fall der Mantelpellets (Arzneiform z. B. Kapsel) ist als weiterer zusätzlicher Vorteil die Möglichkeit der Einstellung beliebiger Freisetzungskinetiken des Wirkstoffes zu nennen (durch Kombination verschiedener Mantelpellets). Dadurch kann eine solche Retardzubereitung entsprechend den Anforderungen eines vorgegebenen Wirkstoffes quasi "maßgeschneidert" werden.

Im Hinblick auf das seit langem bestehende Bedürfnis nach Arzneizubereitungsformen mit lang anhaltender Wirkung ist es mehr als überraschend, daß bisher niemand die einfach herstellbare und sehr effektvolle erfindungsgemäße Mantelarzneiform mit langsam freisetzendem Kern beschrieben oder hergestellt hat. Durch die vorliegende Erfindung kann der Patient in die Lage versetzt werden, das Medikament nur einmal täglich applizieren zu müssen, was insbesondere bei Dauertherapie eine sicherere und angenehmere Behandlungsart darstellt.

Die Kurve der Abb. 1 zeigt für Beispiel 1 das Prinzip der diskontinuierlichen Wirkstofffreisetzung.

Beispiele

Beispiel 1

| | | |
|---|---|---|
| Kern: | Nifedipin 5-50 µm | 20,0 g |
| | Avicel | 34,8 g |
| | Maissstärke | 12,0 g |
| | Milchzucker | 10,0 g |

werden gemischt und granuliert mit

| | |
|---|---|
| Maisstärke | 2 g |
| Tween 80 | 1 g |
| in 30 ml Wasser | |

nach dem Trocknen wird zugemischt

| | |
|---|---|
| Magnesiumstearat | 0,2 g |

Auf einer Tablettenpresse wird zu Kernen mit einem Gewicht von 80 mg, Format 6 mm Durchmesser, verpreßt.
Mantel:

| | |
|---|---|
| Hydroxypropyl-cellulose Typ L | 31,0 g |
| Hydroxypropyl-cellulose Typ M | 106,0 g |
| Lactose fein | 111,5 g |

werden mit 150 ml Wasser granuliert, anschließend wird getrocknet. Zumischen von

| | |
|---|---|
| Magnesiumstearat | 1,5 g |

Auf einer Manteltablettenpresse wird zu Hülltabletten im Format 9 mm Durchmesser verpreßt (Tablettengesamtgewicht 330 mg).

Beispiel 2

Wie Beispiel 1, aber auf die Manteltablette wird eine schnell freisetzende Initialdosis von 10 mg Nifedipin in einem Gemisch von üblichen zur Filmlackierung verwendeten Hilfsstoffen (Hydroxypropylmethylcellulose HPMC, PEG 4000) aufgebracht. Anschließend wird noch ein weiterer Filmlack aufgetragen, der Lichtschutzfunktion hat (HPMC, PEG, Eisenoxid rot).

Beispiel 3

Wie Beispiel 2, aber statt Nifedipin Nisoldipin enthaltend.

Beispiel 4

Wie Beispiel 1, aber statt Nifedipin Nitrendipin enthaltend.

Beispiel 5

Wie Beispiel 4 aber statt Nifedipin die 3 fache Menge Nimodipin enthaltend, Zusätzlich wird die Initialdosis von 30 mg Nimodipin in Form einer festen Lösung (Copräzipitat) aufgepreßt (2 Schicht-Mantel-Tablette, nicht auflackiert), Ein zusätzlicher Lichtschutzlack entfällt.

| Beispiel 6 | | a) | b) | c) |
|---|---|---|---|---|
| Kern: | Nimodipin mikrofein | 78 g | 90 g | 88 g |
| | Hydroxypropylcellulose Typ L | 15 g | - | - |
| | Lactose | 5 g | - | - |
| | Polyvinylpyrrolidon 25 | - | 5 g | 5 g |
| | Natriumlaurylsulfat | 2 g | 2 g | 2 g |
| | $Na_2SO_4$ | - | - | 5 g |
| | Puderzucker | - | 3 g | - |

werden gemischt und durch Pelletierung mit Wasser als Granulierflüssigkeit in sphärische Partikeln im Durchmesserbereich zwischen 0,5-1,5 mm überführt.

Mantel

In einem kontinuierlich arbeitenden Rotorgranulator werden gleichzeitig Nimodipin-Kerne und Mantelpulver gemischt,eindosiert, sowie Wasser als Granulierflüssigkeit eingesprüht. Das Mantel-Pulvergemisch ist wie folgt zusammengesetzt:

Beispiel 6a

| | |
|---|---|
| Reisstärke | 20 % |
| Rizinusöl hydriert | 50 % |
| Hydroxypropylcellulose Typ M | 30 % |

Beispiel 6b

| | |
|---|---|
| Vorverkleisterte Maisstärke | 30 % |
| Hydroxypropylcellulose Typ M | 40 % |
| hydriertes Rizinusöl | 30 % |

Beispiel 6c

| | |
|---|---|
| hydriertes Rizinusöl | 70 % |
| Hydroxypropylcellulose Typ M | 30 % |

Das Mantelpulvergemisth der Beispiele 6a bis 6c wird in folgenden Mengen auf die Kerne aufgetragen:
Beispiel 6a - 250 % des Kerngewichts
Beispiel 6b - 300 % des Kerngewichts
Beispiel 6c - 150 % des Kerngewichts.

## Patentansprüche

### Patentansprüche für folgende Vertragsstaaten: DE, GB, FR, IT, NL, SE, CH, LI, BE, AT, GR

1. Feste Arzneizubereitung mit lang anhaltender Wirkung enthaltend einen schwer löslichen Dihydropyridinwirkstoff der allgemeinen Formel (I)

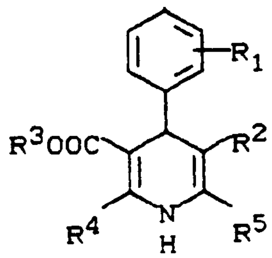

I ,

in welcher

$R^1$ für ein oder zwei gleiche oder verschiedene Substituenten aus der Gruppe Nitro, Halogen, Trifluormethyl oder $OCHF_2$ steht oder in der

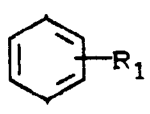 für 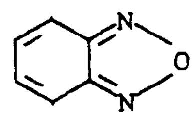 oder

für 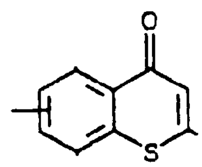 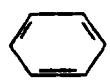 steht,

$R^2$ für eine Nitrogruppe steht oder für den Rest $COOR_6$ steht, wobei

$R_6$ Alkyl mit 1 bis 10 C-Atomen bedeutet, welches gegebenenfalls substituiert ist durch Alkoxy mit 1 bis 4 C-Atomen oder durch ein oder mehrere Halogene

oder wobei

$R^2$ gemeinsam mit $R^5$ für die Lactongruppe $-CO-O-CH_2-$steht,

$R^3$ für Alkyl mit 1 bis 10 C-Atomen steht, welches gegebenenfalls substituiert ist durch Alkoxy mit 1 bis 4 C-Atomen oder durch ein oder mehrere Fluore und

$R^4$ und $R^5$ gleich oder verschieden sind und jeweils für Alkyl mit 1 bis 4 C-Atomen, welches gegebenenfalls durch Hydroxy substituiert ist, stehen,

dadurch gekennzeichnet, daß die Darreichungsform einen Kern-Mantel-Aufbau hat, wobei der Kern mindestens eins der oben genannten Dihydropyridine in langsam freisetzender Form enthält und wobei der um den Kern liegende Mantel keinen Wirkstoff enthält und sich nur langsam auflöst und wobei gegebenenfalls auf dem Mantel zusätzlich eine schnell freisetzende Initialdosis des Wirkstoffs aufgebracht ist.

2. Arzneizubereitung gemäß Anspruch 1, dadurch gekennzeichnet, daß der Kern 10-100 % des gesamten Dihydropyridinwirkstoffs enthält.

3. Arzneizubereitung gemäß Anspruch 1, dadurch gekennzeichnet, daß der Kern 50-100 % des gesamten Dihydropyridinwirkstoffs enthält.

4. Arzneizubereitung gemäß Anspruch 1, dadurch gekennzeichnet, daß sie insgesamt 1-200 mg Dihydropyridinwirkstoffs enthält.

5. Arzneizubereitung gemäß Anspruch 1, dadurch gekennzeichnet, daß der langsam freisetzende Kern den Wirkstoff in retardierter Form enthält.

6. Arzneizubereitung gemäß Anspruch 1, dadurch gekennzeichnet, daß der wirkstofffreie Mantel ein hydrophiles, gelbildendes Polymer und gegebenenfalls weitere übliche Hilfsstoffe enthält.

7. Arzneizubereitung gemäß Anspruch 1, dadurch gekennzeichnet, daß der wirkstofffreie Mantel als hydrophile gelbildende Polymere modifizierte Stärke und/oder celluloseartige Substanzen wie Methylcellulose, Hydroxypropylmethylcellulose, Hydroxypropylcellulose oder Natriumcarboxymethylcellulose enthält.

**Patentanspruch für folgenden Vertragsstaat: ES**

1. Verfahren zur Herstellung von festen Arzneisubereitungen mit lang anahltender Wirkung enthaltend einen schwer löslichen Dihydropyridinwirkstoff der allgemeinen Formel I

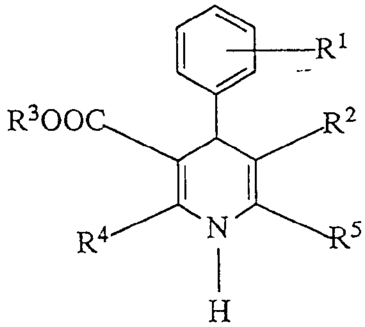

(I)

in welcher

$R^1$ für ein oder zwei gleiche oder verschiedene Substituenten aus der Gruppe Nitro, Halogen, Trifluormethyl oder $OCHF_2$ steht oder in der

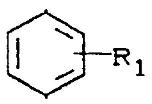 für 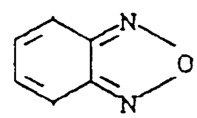 oder

für 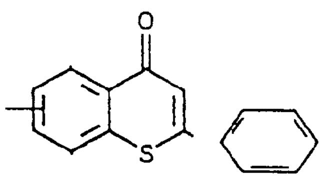 steht,

$R^2$ für eine Nitrogruppe steht oder für den Rest $COOR_6$ steht, wobei

$R_6$ Alkyl mit 1 bis 10 C-Atomen bedeutet, welches gegebenenfalls substituiert ist durch Alkoxy mit 1 bis 4 C-Atomen oder durch ein oder mehrere Halogene

oder wobei

$R^2$ gemeinsam mit $R^5$ für die Lactongruppe $-CO-O-CH_2-$steht,

$R^3$ für Alkyl mit 1 bis 10 C-Atomen steht, welches gegebenenfalls substituiert ist durch Alkoxy mit 1 bis 4 C-Atomen oder durch ein oder mehrere Fluore und

$R^4$ und $R^5$ gleich oder verschieden sind und jeweils für Alkyl mit 1 bis 4 C-Atomen, welches gegebenenfalls durch Hydroxy substituiert ist, stehen,

dadurch gekennzeichnet, daß die Darreichungsform einen Kern-Mantel-Aufbau hat, wobei der Kern mindestens eins der oben genannten Dihydropyridine in langsam freisetzender Form enthält und wobei der um den Kern liegende Mantel keinen Wirkstoff enthält und sich nur langsam auflöst und wobei gegebenenfalls auf dem Mantel zusätzlich eine schnell freisetzende Initialdosis des Wirkstoffs aufgebracht ist, indem man nach üblichen Methoden eine geeignete Wirkstoffdosis, gegebenenfalls unter Verwendung von Hilfs- und Trägerstoffen in einen retardierten Kern überführt und diesen Kern nach üblichen Methoden unter Verwendung von hydrophilen gelbildenden Polymeren und üblichen Hilfsstoffen mit einem wirkstofffreien Mantel umgibt, auf den gegebenenfalls noch eine schnell freisetzende Initialdosis des Wirkstoffs aufgebracht wird.

## Claims

### Claims for the following Contracting States: DE, GB, FR, IT, NL, SE, CH, LI, BE, AT, GR

1. Solid medicament preparation having long-lasting action containing a sparingly soluble dihydropyridine active compound of the general formula (I)

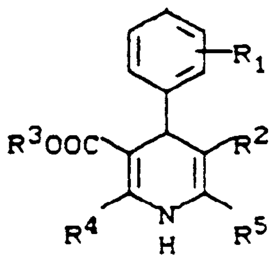

I,

in which

$R^1$ represents one or two identical or different substituents from the group comprising nitro, halogen, trifluoromethyl or $OCHF_2$ or in which

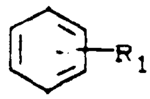

represents

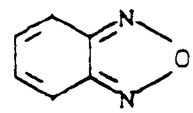

or

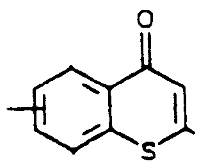

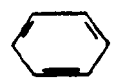

$R^2$ represents a nitro group or the radical $COOR_6$, where

$R_6$ denotes alkyl having 1 to 10 C atoms which is optionally substituted by alkoxy having 1 to 4 carbon atoms or by one or more halogens

or where

$R^2$ together with $R^5$ represents the lactone group $-CO-O-CH_2-$,

$R^3$ represents alkyl having 1 to 10 C atoms, which is optionally substituted by alkoxy having 1 to 4 C atoms or by one or more fluorines and

$R^4$ and $R^5$ are identical or different and in each case represent alkyl having 1 to 4 C atoms, which is optionally substituted by hydroxyl,

characterised in that the administration form has a core-coating construction where the core contains at least one of the abovementioned dihydropyridines in slow-release form and where the coating situated around the core contains no active compound and only dissolves slowly and where, if desired, a rapid-release initial dose of the active compound is additionally applied to the coating.

2. Medicament preparation according to Claim 1, characterised in that the core contains 10-100% of the total dihydropyridine active compound.

3. Medicament preparation according to Claim 1, characterised in that the core contains 50-100% of the total dihydropyridine active compound.

4. Medicament preparation according to Claim 1, characterised in that it contains 1-200 mg of dihydropyridine active compound in total.

5. Medicament preparation according to Claim 1, characterised in that the slow-release core contains the active compound in delayed release form.

6. Medicament preparation according to Claim 1, characterised in that the active compound-free coating contains a hydrophilic, gel-forming polymer and, if desired, further customary auxiliaries.

7. Medicament preparation according to Claim 1, characterised in that the active compound-free coating contains modified starch and/or cellulose-like substances such as methylcellulose, hydroxypropylmethylcellulose, hydroxypropylcellulose or sodium carboxymethylcellulose as hydrophilic gel-forming polymers.

## Claims for the following Contracting State: ES

1. Process for the preparation of solid medicament preparations having long-lasting action containing a sparingly soluble dihydropyridine active compound of the general formula I

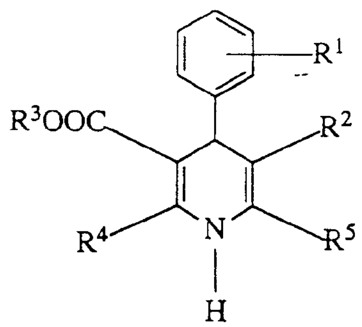

(I)

in which

$R^1$ represents one or two identical or different substituents from the group comprising nitro, halogen, trifluoromethyl or $OCHF_2$ or in which

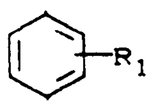

represents

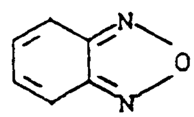

or

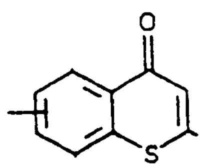

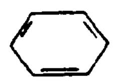

$R^2$ represents a nitro group or the radical $COOR_6$, where

$R_6$ denotes alkyl having 1 to 10 C atoms which is optionally substituted by alkoxy having 1 to 4 carbon atoms or by one or more halogens

or where

$R^2$ together with $R^5$ represents the lactone group $-CO-O-CH_2-$,

$R^3$ represents alkyl having 1 to 10 C atoms, which is optionally substituted by alkoxy having 1 to 4 C atoms or by one or more fluorines and

$R^4$ and $R^5$ are identical or different and in each case represent alkyl having 1 to 4 C atoms, which is optionally substituted by hydroxyl,

characterised in that the administration form has a core-coating construction where the core contains at least one of the abovementioned dihydropyridines in slow-release form and where the coating situated around the core contains no active compound and only dissolves slowly and where, if desired, a rapid-release initial dose of the active compound is additionally applied to the coating, by converting a suitable dose of active compound by customary methods, if desired using auxiliaries and excipients, into a sustained-release core and surrounding this core by customary methods, using hydrophilic, gel-forming polymers and customary auxiliaries, with an active compound-free coating to which, if desired, a rapid-release initial dose of the active compound is additionally applied.

## Revendications

### Revendications pour les Etats contractants suivants: DE, GB, FR, IT, NL, SE, CH, LI, BE, AT, GR

1. Composition médicamenteuse solide à activité persistant pendant de longues durées, contenant une substance active peu soluble du type dihydropyridine répondant à la formule générale I

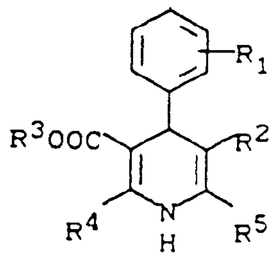

I,

dans laquelle

$R^1$ représente un ou deux substituants identiques ou différents choisis parmi les groupes nitro, les halogènes, les groupes trifluorométhyle, ou $OCHF_2$, ou bien

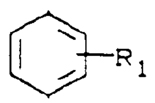 représente 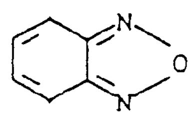 ou

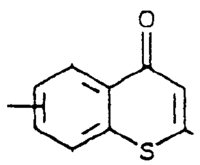

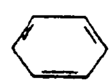

$R^2$ représente un groupe nitro ou $COOR_6$ dans lequel

$R_6$ représente un groupe alkyle en $C_1$-$C_{10}$ éventuellement substitué par un groupe alcoxy en $C_1$-$C_4$ ou par un ou plusieurs atomes d'halogène, ou bien

$R^2$ forme avec $R^5$ le groupe lactone $-CO-O-CH_2$,

$R^3$ représente un groupe alkyle en $C_1$-$C_{10}$ éventuellement substitué par un groupe alcoxy en $C_1$-$C_4$ ou par un ou plusieurs atomes de fluor, et

$R^4$ et $R^5$ ayant des significations identiques ou différentes, représentent chacun un groupe alkyle en $C_1$-$C_4$ éventuellement substitué par un groupe hydroxy,

caractérisée en ce que la forme de présentation est à structure noyau-enveloppe, le noyau contenant au moins une des dihydropyridines mentionnées ci-dessus sous une forme à libération lente et l'enveloppe entourant le noyau ne contenant pas de substance active et ne se dissolvant que lentement, une dose initiale à libération rapide de la substance active étant en outre appliquée le cas échéant sur l'enveloppe.

2. Composition médicamenteuse selon revendication 1, caractérisée en ce que le noyau contient de 10 à 100 % de la substance active totale du type dihydropyridine.

3. Composition médicamenteuse selon revendication 1, caractérisée en ce que le noyau contient de 50 à 100 % de la totalité de la substance active du type dihydropyridine.

4. Composition médicamenteuse selon revendication 1, caractérisée en ce qu'elle contient au total de 1 à 200 mg de substance active du type dihydropyridine.

5. Composition médicamenteuse selon revendication 1, caractérisée en ce que le noyau à libération lente contient la substance active sous une forme retard.

6. Composition médicamenteuse selon revendicaiton 1, caractérisée en ce que l'enveloppe, exempte de substance active, contient un polymère hydrophile gélifiable et le cas échéant d'autres produits auxiliaires usuels.

7. Composition médicamenteuse selon revendication 1, caractérisée en ce que l'enveloppe exempte de substance active contient en tant que polymères hydrophiles gélifiables des amidons modifiés et/ou des substances de nature cellulosique comme la méthylcellulose, l'hydroxypropylméthylcellulose, l'hydroxypropylcellulose ou la carboxyméthylcellulose sodique.

**Revendication pour l'Etat contractant suivant: ES**

1. Procédé de préparation de compositions médicamenteuses solides à activité persistant pendant de longues durées, contenant une substance active peu soluble du type dihydropyridine de formule générale :

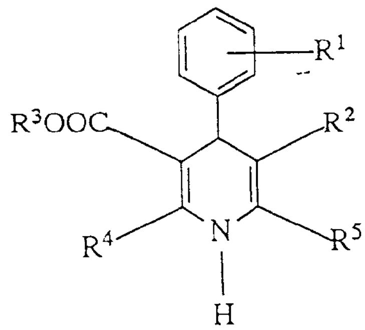

(I)

dans laquelle

$R^1$ représente un ou deux substituants identiques ou différents choisis parmi les groupes nitro, les halogènes, les groupes trifluorométhyle ou $OCHF_2$, ou bien

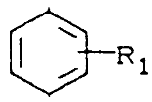 représente 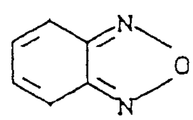 ou

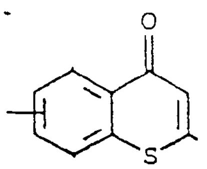 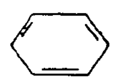

$R^2$ représente un groupe nitro ou $COOR_6$ dans lequel

$R_6$ représente un groupe alkyle en $C_1$-$C_{10}$ éventuellement substitué par un groupe alcoxy en $C_1$-$C_4$ ou par un ou plusieurs atomes d'halogènes,

ou bien

$R^2$ forme avec $R^5$ le groupe lactone $-CO-O-CH_2-$,

$R^3$ représente un groupe alkyle en $C_1$-$C_{10}$ éventuellement substitué par un groupe alcoxy en $C_1$-$C_4$ ou par un ou plusieurs atomes de fluor, et

$R^4$ et $R^5$ ayant des significations identiques ou différents, représentent chacun un groupe alkyle en $C_1$-$C_4$ éventuellement substitué par un groupe hydroxy,

caractérisé en ce que la forme de présentation est à structure noyau-enveloppe, le noyau contenant au moins une des dihydropyridines mentionnées ci-dessus sous une forme à libération lente, l'enveloppe enrobant le noyau ne contenant pas de substance active et ne se dissolvant que lentement, une dose initiale à libération rapide de la substance active étant éventuellement appliquée en outre sur l'enveloppe, selon lequel on met une dose appropriée de substance active, par des techniques usuelles, éventuellement en utilisant des produits auxiliaires et véhicules, sous forme de noyau retardé et on enrobe ce noyau par des techniques usuelles, à l'aide de polymères hydrophiles gélifiables et de produits auxiliaires usuels, par une enveloppe exempte de substance active sur laquelle le cas échéant on applique encore une dose initiale à libération rapide de la substance active.

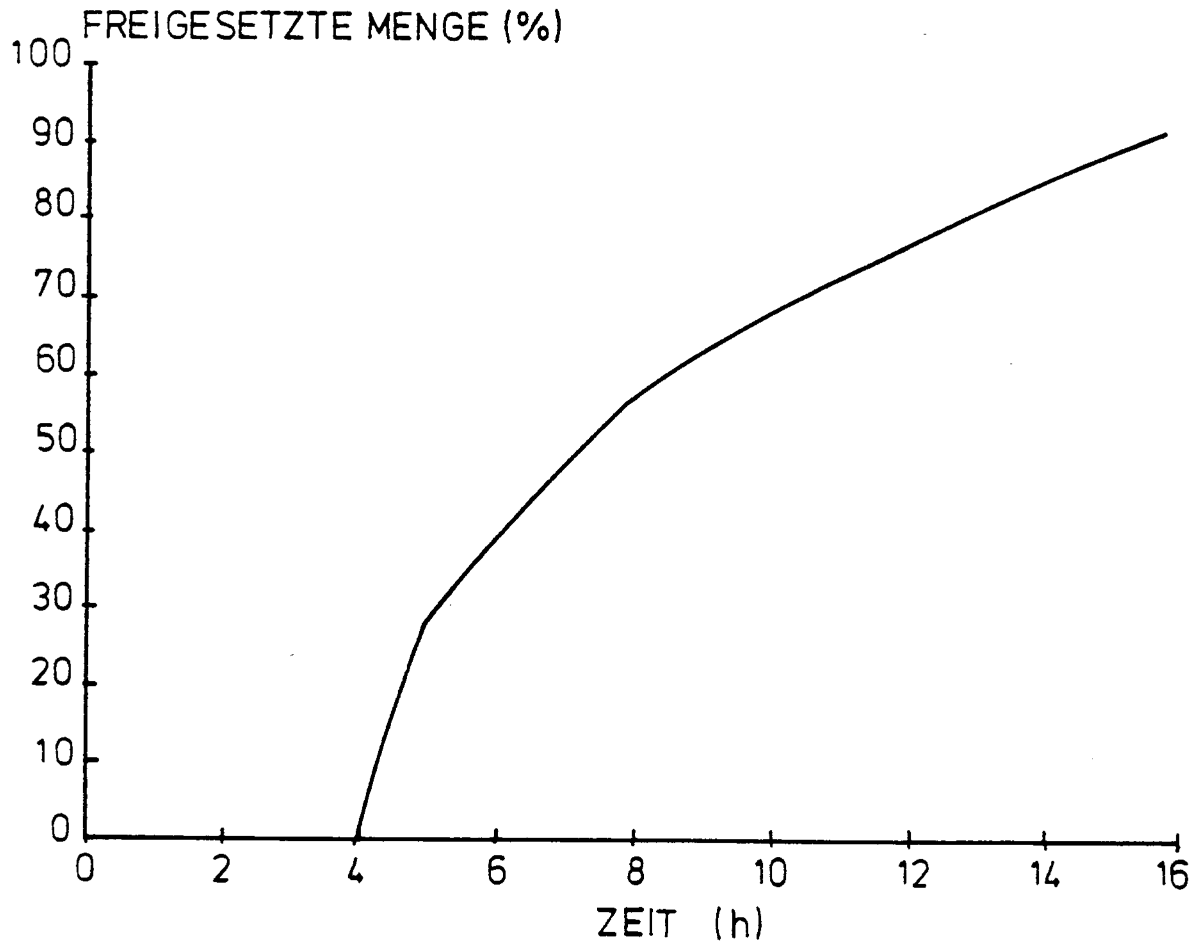
FREIGESETZTE MENGE (%)
100
90
80
70
60
50
40
30
20
10
0
0
2
4
6
8
10
12
14
16
ZEIT (h)

FIG.1